(19) Europäisches Patentamt
European Patent Office
Office européen des brevets

(11) Veröffentlichungsnummer: 0 236 829
A2

# (12) EUROPÄISCHE PATENTANMELDUNG

(21) Anmeldenummer: 87102518.5

(22) Anmeldetag: 23.02.87

(51) Int. Cl.⁴: **C07D 239/34** , C07D 239/52 , C07D 239/56 , A01N 47/30

(30) Priorität: 07.03.86 DE 3607628

(43) Veröffentlichungstag der Anmeldung: 16.09.87 Patentblatt 87/38

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Schwamborn, Michael, Dr.
von-Lohe-Strasse 9
D-5000 Köln 80(DE)
Erfinder: Baasner, Bernd, Dr.
Hamberger Strasse 27d
D-5090 Leverkusen 3(DE)
Erfinder: Schmidt, Robert R., Dr.
Im Waldwinkel 110
D-5060 Bergisch-Gladbbach 2(DE)
Erfinder: Santel, Hans-Joachim, Dr.
Grünstrasse 9a
D-5090 Leverkusen 1(DE)
Erfinder: Strang, Harry, Dr.
Unterdorfstrasse 6A
D-4000 Düsseldorf 31(DE)

## (54) 2-Trifluormethyl-5-pyrimidinyloxy-phenyl-harnstoffe und ein Verfahren zu ihrer Herstellung.

(57) Die Erfindung betrifft 2-Trifluormethyl-5-pyrimidinyloxy-phenyl-harnstoffe der Formel (I),

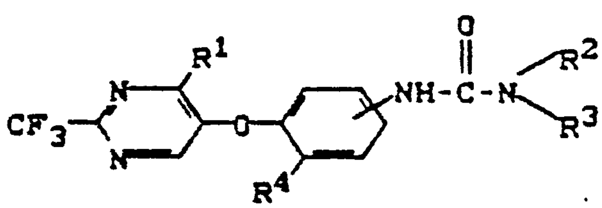

(I)

in welcher
$R^1$ für Wasserstoff, Alkoxy oder Alkylthio steht,
$R^2$ für Alkyl steht,
$R^3$ für Wasserstoff, Alkyl oder Alkoxy steht und
$R^4$ für Wasserstoff, Alkyl oder Halogen steht,
ein Verfahren zu ihrer Herstellung und ihre Verwendung als Herbizide.

EP 0 236 829 A2

## 2-Trifluormethyl-5-pyrimidinyloxy-phenyl-harnstoffe und ein Verfahren zu ihrer Herstellung

Die vorliegende Erfindung betrifft neue 2-Trifluormethyl-5-pyrimidinyloxy-phenyl-harnstoffe, ein Verfahren zu ihrer Herstellung und ihre Verwendung als Herbizide.

Es ist bereits bekannt, daß bestimmte Phenylharnstoffe herbizide Eigenschaften besitzen (vgl. z. B. US 4 001 234 und DE-AS 1.142.251). So kann z. B. der N,N-Dimethyl-N'-[4-(2-chlor-4-pyrimidinyloxy)-phenyl]-harnstoff als Herbizid eingesetzt werden. Die Wirkung dieses Stoffes ist jedoch nicht immer ausreichend.

Es wurden nun neue 2-Trifluormethyl-5-pyrimidinyloxy-phenyl-harnstoffe der Formel (I),

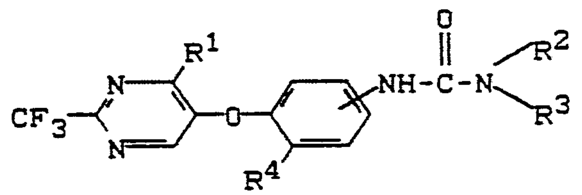

(I)

in welcher
$R^1$ für Wasserstoff, Alkoxy oder Alkylthio steht,
$R^2$ für Alkyl steht,
$R^3$ für Wasserstoff, Alkyl oder Alkoxy steht und
$R^4$ für Wasserstoff, Alkyl oder Halogen steht,
aufgefunden.

Weiterhin wurde gefunden, daß man die neuen 2-Trifluormethyl-5-pyrimidinyloxy-phenyl-harnstoffe der allgemeinen Formel (I) erhält, wenn man Pyrimidinderivate der allgemeinen Formel (II),

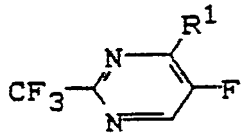

(II)

in welcher
$R^1$ die oben angegebene Bedeutung hat,
mit substituierten Harnstoffderivaten der allgemeinen Formel (III),

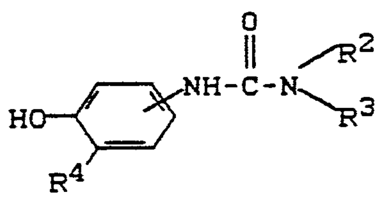

(III)

in welcher
$R^2$, $R^3$ und $R^4$ die oben angegebene Bedeutung haben,
gegebenenfalls in Gegenwart eines Säurebindemittels und gegebenenfalls in Gegenwart eines Verdünnungsmittels umsetzt.

Schließlich wurde gefunden, daß die neuen 2-Trifluormethyl-5-pyrimidinyloxy-phenyl-harnstoffe der allgemeinen Formel (I) starke herbizide Eigenschaften aufweisen.

Überraschenderweise sind die erfindungsgemäßen Wirkstoffe der allgemeinen Formel (I) bei gleicher Kulturpflanzenverträglichkeit deutlich wirksamer als die vorbekannten Phenylharnstoffe wie z. B. der vorbekannte N,N-Dimethyl-N'-[4-(2-chlor-4-pyrimidinyloxy)-phenyl]-harnstoff (bekannt aus US 4 001 234).

Die erfindungsgemäßen neuen Phenylharnstoffe der allgemeinen Formel (I) zeichnen sich gegenüber vorbekannten Phenylharnstoffen strukturell insbesondere durch einen gegebenenfalls in 6-Stellung substituierten 2-Trifluormethyl-5-pyrimidinyloxy-Substituenten aus.

Überraschenderweise ist bei dem erfindungsgemäßen Verfahren der 5-Fluorsubstituent der Pyrimidinderivate der Formel (II) unter relativ milden Bedingungen einer nucleo philen Substitution durch die Phenylharnstoffe der allgemeinen Formel (III) zugänglich. Dies steht im deutlichen Gegensatz zur sonst unmöglichen bzw. in einigen Fällen sehr schweren Substituierbarkeit der Chlor-oder Fluorsubstituenten in anderen 5-Halogenpyrimidinderivaten (vgl.: A. Weissberger, The Chemistry opf Heterocyclic Compounds, The Pyrimidines, S. 210 ff, Interscience New York 1962, The Pyrimidines Suppl. I, S. 156 ff (1970), The Pyrimidines Suppl. II (1985))

So ist der 5-Halogensubstituent in Tetrachlor-oder Tetrafluorpyrimidin nur bei hohen Temperaturen (T >220°C) z.B. mit Aminen substituierbar (vgl.: R.E.Banks et al. J.Chem. Soc. 1822 ff. (1967); H.Schroeder et al. J. Org. Chem. 2580 ff. (1962)) und 5-Chlor-bzw. 5-chlor-2-methylpyrimidin reagieren mit Stickstoff-Nucleophilen bevorzugt unter Bildung von 4-N-substituierten Pyrimidinen und nicht zu 5-N-substituierten Pyrimidinderivaten (vgl.: T.Kaufmann et al. Chem. Ber. 102 1177 ff.; Schwan et al. J. Org. Chem. 29, 941 - (1964)).

Die vorliegende Erfindung betrifft sowohl die neuen 2-Trifluormethyl-5-pyrimidyloxy-phenyl-harnstoffe der Formel (I) als auch das erfinderische Verfahren zu ihrer Herstellung.

Von den erfindungsgemäßen 2-Trifluormethyl-5-pyrimidinyloxy-phenyl-harnstoffen der Formel (I) sind bevorzugt diejenigen, in denen

$R^1$ für Wasserstoff, Alkoxy mit 1-8 C-Atomen oder Alkylthio mit 1-8 C-Atomen steht,

$R^2$ für Alkyl mit 1-6 C-Atomen steht,

$R^3$ für Wasserstoff, Alkyl mit 1-6 C-Atomen oder für Alkoxy mit 1-6 C-Atomen steht und

$R^4$ für Wasserstoff, für Alkyl mit 1-6 C-Atomen oder für Fluor, Chlor, Brom oder Iod steht.

Aus dieser Stoffgruppe sind solche Verbindungen der Formel (I) besonders bevorzugt, in denen

$R^1$ für Wasserstoff, Alkoxy mit 1-4 C-Atomen oder Alkylthio mit 1-4 C-Atomen steht,

$R^2$ für Alkyl mit 1-4 C-Atomen steht,

$R^3$ für Wasserstoff, für Alkyl mit 1-4 C-Atomen oder für Alkoxy mit 1-4 C-Atomen steht und

$R^4$ für Wasserstoff, für Alkyl mit 1-4 C-Atomen oder für Fluor oder Chlor steht.

Verwendet man beispielsweise 5-Fluor-2-trifluormethylpyrimidin und N,N-Dimethyl-N'-(3-hydroxyphenyl)-harnstoff als Ausgangsstoffe, so kann der Reaktionsablauf des erfindungsgemäßen Verfahrens durch das folgende Formelschema zusammenfassend wiedergegeben werden:

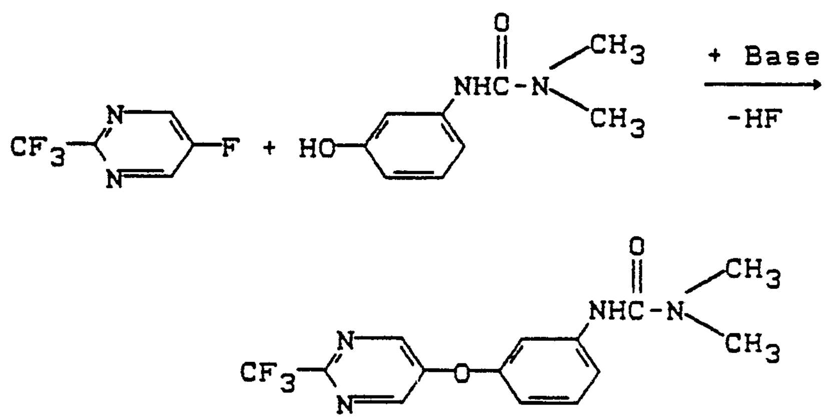


Die als Ausgangsstoffe eingesetzten Pyrimidinderivate sind durch die Formel (II) allgemein definiert. In dieser Formel steht $R^1$ vorzugsweise bzw. besonders bevorzugt für diejenigen Reste, die schon bei der Beschriebung der erfindungsgemäßen Stoffe der Formel (I) als bevorzugt bzw. besonders bevorzugt für diesen Substituenten genannt wurden.

Die Pyrimidinderivate der Formel (II) sind zum Teil bekannt oder können nach gängigen Verfahren der organischen Chemie erhalten werden (vgl. Herstellungsbeispiele).

Die weiterhin als Ausgangsstoffe verwendeten Phenylharnstoffe sind durch die Formel (III) allgemein definiert. In dieser Formel (III) stehen $R^2$, $R^3$ und $R_4$ bevorzugt bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden.

Die Phenylharnstoffe der allgemeinen Formel (III) sind bekannt oder lassen sich nach bekannten Verfahren in analo ger Weise herstellen (vgl. z. B. Neth. Appl. 6,503 645, US 4 001 234 sowie Houben-Weyl, Methoden der organischen Chemie Bd. E 4, Thieme Verlag, Stuttgart, 1983, 334 ff.)

Als Verdünnungsmittel zur Durchführung des erfindungsgemäßen Verfahrens kommen inerte organische Lösungsmittel sowie gegebenenfalls Wasser in Frage.

Hierzu gehören insbesondere aliphatische oder aromatische, gegebenenfalls halogenierte Kohlenwasserstoffe, wie beispeilsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl-oder diethylether; Ketone, wie Aceton oder Butanon; Nitrile wie Acetonitril oder Propionitril; Amide, wie Dimethylformamid, Dimethylacetamid, N-Methylformanilid, N-Methyl-pyrrolidon oder

Hexamethylphosphorsäuretriamid oder Ester, wie Essigsäureethylester, Bevorzugte Lösungsmittel sind Ether wie Diethylether, Dioxan, Tetrahydrofuran oder Ethylenglykoldimethyl-oder diethylether, Ketone, wie Aceton oder Butanon, Nitrile wie Acetonitril oder Propionitril, Amide wie Dimethylformamid, N-Methylpyrrolidon oder Hexamethylphosphorsäuretriamid.

Das erfindungsgemäße Verfahren wird gegebenenfalls in Gegenwart eines Säurebindemittels durchgeführt.

Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin oder N,N-Dimethylaminopyridin. Bevorzugt verwendet man Alkalimetallhydroxide, Natriumhydrid, Alkalimetallcarbonate oder tertiäre Amine.

Die Umsetzung wird im Druckbereich von 1 bis etwa 10 bar durchgeführt.

Bei der Durchführung des erfindungsgemäßen Verfahrens setzt man auf 1 Mol Pyrimidin der Formel - (II) im allgemeinen 1-2 Mol, vorzugsweise 1 bis 1,1 Mol Phenylharnstoff der Formel (III) und 1 bis 2 Mol, vorzugsweise 1 bis 1,2 Mol Säurebindemittel ein. Besonders bevorzugt wird unter Einsatz stöchiometrischer Molverhältnisse gearbeitet.

Die Reaktionstemperaturen können bei dem erfindungsgemäßen Verfahren in einem größeren Bereich variiert werden. Das Verfahren wird im allgemeinen bei Temperaturen von -30 bis +250°C, vorzugsweise von -20 bis +150°C durchgeführt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernis, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicai, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoff ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Ünkrautbekämpfung in einjährigen Kulturen eingesetzt werden.

Die erfindungsgemäßen Wirkstoffe eignen sich zur Bekämpfung von mono-und dikotylen Unkräutern im Vor-und Nachauflaufverfahren in mono-und dikotylen Kulturen wie z. B. Mais, Reis, Soja und Getreide - (insbesondere Weizen und Gerste). Im Nachauflaufverfahren lassen sich die erfindungsgemäßen Stoffe insbesondere gegen dikotyle Unkräuter einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, als flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff proHektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

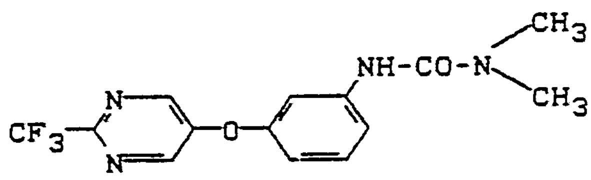

N,N-Dimethyl-N'-[3-(2-trifluormethyl-5-pyrimidinyloxy)-phenyl]-harnstoff

9 g (0,05 mol) N,N-Dimethyl-N'-[3-hydroxyphenyl]-harnstoff werden in Acetonitril unter Zugabe von 20 g $K_2CO_3$ 30 Minuten zum Rückfluß erhitzt. Hierzu tropft man eine Lösung von 8,3 g (0,05 mol) 2-Trifluormethyl-5-fluorpyrimidin und erhitzt 4 Stunden unter Rückfluß. Das Reaktionsgemisch wird in 2 l Eiswasser eingerührt, abgesaugt und getrocknet. Man erhält 13 g (80,2 % der Theorie) des gewünschten Harnstoffs. Fp.: 140°C.

Gemäß Beispiel 1 und gemäß den allgemeinen Angaben zur Herstellung erhält man die in der nachfolgenden Tabelle 1 genannten Harnstoffderivate der allgemeinen Formel (I):

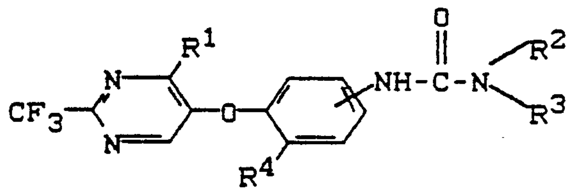

(I)

Tabelle 1

| Bsp. Nr. | $R^1$ | $R^2$ | $R^3$ | $R^4$ | Verknüpfung* | Schmelzpunkt/° C |
|---|---|---|---|---|---|---|
| 2 | H | $-CH_3$ | $-CH_3$ | H | para | 180 |
| 3 | H | $-CH_3$ | $-CH_3$ | -Cl | meta | 167 |
| 4 | H | $-CH_3$ | -H | $-CH_3$ | para | 203 |
| 5 | H | $-CH_3$ | $-CH_3$ | -Cl | para | 223 |
| 6 | H | $-CH_3$ | -H | H | meta | 156 |
| 7 | H | $-CH_3$ | $-CH_3$ | $-CH_3$ | meta | 158 |
| 8 | H | $-CH_3$ | $-OCH_3$ | H | meta | 71- 74 |
| 9 | H | $-C_4H_9$-n | $-CH_3$ | H | meta | Öl |
| 10 | $-OCH_3$ | $-CH_3$ | $-CH_3$ | H | meta | 118 |
| 11 | H | $-C_4H_9$-t | -H | H | meta | 147-149 |
| 12 | H | $-C_3H_7$-i | $-CH_3$ | H | meta | 112-113 |
| 13 | $-SCH_3$ | $-CH_3$ | $-CH_3$ | H | meta | 137 |
| 14 | $-OC_3H_7$-i | $CH_3$ | $-CH_3$ | H | meta | Öl |

*Verknüpfung: Stellung der Pyrimidinyleinheit und der Harnstoffeinheit am Phenylring zueinander.

Herstellung der Ausgangsstoffe

Die als Ausgangsstoffe verwendeten Pyrimidinderivate (II-1) bis (II-5) sind zum Teil bekannt oder können nach bekannten Verfahren der organischen Chemie erhalten werden (vgl. A. Weissberger, The Chemistry of Heterocyclic Compounds: The Pyrimdines S. 201 ff., Interscience New York 1962)

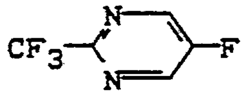

(II-1): bekannt aus EP 154 122

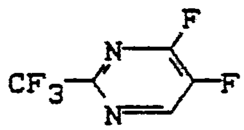

(II-2): bekannt aus EP 154 122

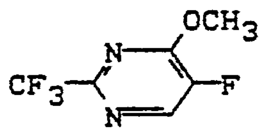

(II-3): aus (II-2) durch Methanolyse
Kp: 54-56° C/13 mbar,
$n_D^{20}$ : 1,4148

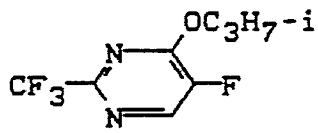

(II-4): aus (II-2) durch Isopropanolyse
Kp: 110° C/26 mbar,

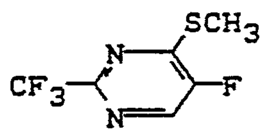

(II-5): aus (II-2) durch Umsetzung mit $NaSCH_3$
Kp: 90° C/26 mbar,

Anwendungsbeispiele

In den folgenden Anwendungsbeispielen wurden die nachstehend aufgeführten Verbindungen als Vergleichssubstanzen herangezogen:

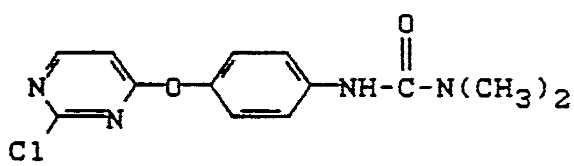

(A)

bekannt aus US-P-4 001 234 (Beispiel 1)

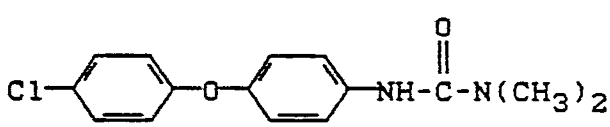

(B)

bekannt aus DE-AS 1.142.251

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei einer Aufwandmenge von beispielsweise 0,5 kg/ha zeigen z. B. die Verbindungen gemäß den Herstellungsbeispielen 1 und 2 in diesem Test bei gleicher Kulturpflanzenverträglichkeit (0 % Schädigung bei Weizen) eine deutlich bessere herbizide Wirksamkeit (80 -100 % Schädigung) gegen mono-und dikotyle Unkräuter wie Datura, Galinsoga, Panicum, Setaria, Stellaria und Matricaria, als die Vergleichssubstanz (A) - (0 -20 % Schädigung) und die Vergleichssubstanz (B) (0 bis 30 % Schädigung).

Beispiel B

Post-emergence-Test

Lösungsmittel: 5 Gewichtsteile Aceton
Emulgator: 1 Gewichtsteil Alkylarylopolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen z.B. die Verbindungen gemäß den Herstellungsbeispielen 1, 6 und 9 bei gleicher Kulturpflanzenverträglichkeit im Mais eine deutlich bessere herbizide Wirksamkeit gegen mono-und dikotyle Unkräuter wie beispielsweise Chenopodium, Stellaria, Echinochloa und Setaria als die Vergleichssubstanz - (A).

Weiterhin zeigen in diesem Text z.B. die Verbindungen gemäß den Herstellungsbeispielen 1, 6 und 9 sowohl eine deutlich bessere Kulturpflanzenverträglichkeit im Mais als auch eine deutlich bessere herbizide Wirksamkeit gegen Unkräuter wie beispielsweise Ipomoea, Abutilon, Datura und Galinsoga als die Vergleichssubstanz (B).

## Ansprüche

1. 2-Trifluormethyl-5-pyrimidinyloxy-phenyl-harnstoffe der Formel (I),

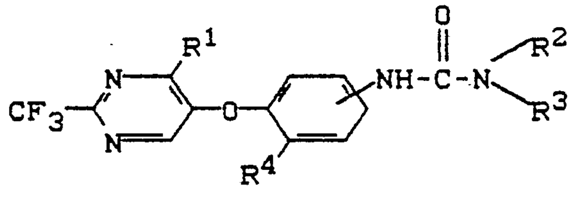

(I)

in welcher
$R^1$ für Wasserstoff, Alkoxy oder Alkylthio steht,
$R^2$ für Alkyl steht,
$R^3$ für Wasserstoff, Alkyl oder Alkoxy steht und

$R^4$ für Wasserstoff, Alkyl oder Halogen steht.

2. 2-Trifluormethyl-5-pyrimidinyloxy-phenyl-harnstoffe der Formel (I) gemäß Anspruch 1, in welcher
$R^1$ für Wasserstoff, Alkoxy mit 1-8 C-Atomen oder Alkylthio mit 1-8 C-Atomen steht,
$R^2$ für Alkyl mit 1-6 C-Atomen steht,
$R^3$ für Wasserstoff, Alkyl mit 1-6 C-Atomen oder für Alkoxy mit 1-6 C-Atomen steht und
$R^4$ für Wasserstoff, für Alkyl mit 1-6 C-Atomen oder für Fluor, Chlor, Brom oder Iod steht.

3. 2-Trifluormethyl-5-pyrimidinyloxy-phenyl-harnstoffe der Formel (I) gemäß Anspruch 1, in welcher
$R^1$ für Wasserstoff, Alkoxy mit 1-4 C-Atomen oder Alkylthio mit 1-4 C-Atomen steht,
$R^2$ für Alkyl mit 1-4 C-Atomen steht,
$R^3$ für Wasserstoff, für Alkyl mit 1-4 C-Atomen oder für Alkoxy mit 1-4 C-Atomen steht und
$R^4$ für Wasserstoff, für Alkyl mit 1-4 C-Atomen oder für Fluor oder Chlor steht.

4. Verfahren zur Herstellung von 2-Trifluormethyl-5-pyrimidinyloxy-phenyl-harnstoffen der Formel (I)

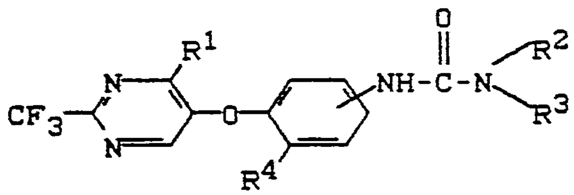

(I)

in welcher
$R^1$ für Wasserstoff, Alkoxy oder Alkylthio steht,
$R^2$ für Alkyl steht,
$R^3$ für Wasserstoff, Alkyl oder Alkoxy steht und
$R^4$ für Wasserstoff, Alkyl oder Halogen steht,
dadurch gekennzeichnet, daß man Pyrimidinderivate der Formel (II),

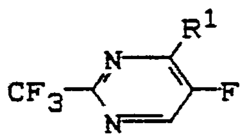

(II)

in welcher
$R^1$ die oben angegebene Bedeutung hat,
mit substituierten Harnstoffderivaten der Formel (III),

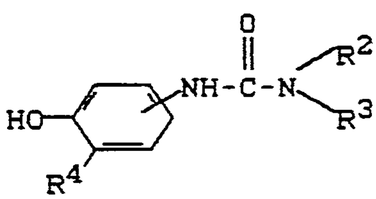

(III)

in welcher
$R^2$, $R^3$ und $R^4$ die oben angegebene Bedeutung haben,
gegebenenfalls in Gegenwart eines Säurebindemittels und gegebenenfalls in Gegenwart eines Verdünnungsmittels umsetzt.

5. Herbizide Mittel, gekennzeichnet durch einen Gehalt an mindestens einem 2-Trifluormethyl-5-pyrimidinyloxy-phenyl-harnstoff der Formel (I) gemäß den Ansprüche 1 bis 4.

6. Verfahren zur Bekämpfung von Unkräutern, dadurch gekennzeichnet, daß man 2-Trifluormethyl-5-pyrimidinyloxy-phenyl-harnstoffe der Formel (I) gemäß den Ansprüchen 1 bis 4 auf die Unkräuter und/oder ihren Lebensraum einwirken läßt.

7. Verwendung von 2-Trifluormethyl-5-pyrimidinyloxy-phenyl-harnstoffen der Formel (I) gemäß den Ansprüchen 1 bis 4 zur Bekämpfung von Unkräutern.

8. Verfahren zur Herstellung von herbiziden Mitteln, dadurch gekennzeichnet, daß man 2-Trifluormethyl-5-pyrimidinyloxy-phenyl-harnstoffe der Formel (I) gemäß den Ansprüchen 1 bis 4 mit Streckmitteln und/oder oberflächenaktiven Substanzen vermischt.